(19) Europäisches Patentamt
European Patent Office
Office européen des brevets

(11) Publication number: **0 608 570 A1**

(12) # EUROPEAN PATENT APPLICATION

(21) Application number: **93121138.7**

(51) Int. Cl.5: **C07D 475/04**, G01N 33/50

(22) Date of filing: **30.12.93**

(30) Priority: **30.12.92 DE 4244561**
**30.12.92 DE 9217853 U**

(43) Date of publication of application:
**03.08.94 Bulletin 94/31**

(84) Designated Contracting States:
**AT CH FR GB IT LI**

(71) Applicant: **EDUCATIONAL FOUNDATION FUJITA GAKUEN**
**12-1, Minamiyakata,**
**Sakae-cho**
**Toyoake, Aichi(JP)**

(72) Inventor: **Sugimoto, Takashi**
**30-22 Takane,**
**Ushida-cho**
**Chiryu, Aichi(JP)**
Inventor: **Ogiwara, Shoji**
**No. 102 Torami-so,**
**1-22-3 Yutaka**
**Minami-ku, Nagoya, Aichi(JP)**
Inventor: **Hidaka, Hiroyoshi**
**1101-1-5-104 Hachimanyama,**
**Tenpaku-ku**
**Nagoya, Aichi(JP)**
Inventor: **Teradaira, Ryo, c/o Inst. for Comprehensive Med. Science,**
**Fujita Health Univ.,**
**1-98 Dengakugakubo**
**Kutsukake-cho, Toyoake, Aichi(JP)**
Inventor: **Fujita, Keisuke, c/o Inst. for Comprehensive Med. Science,**
**Fujita Health Univ.,**
**1-98 Dengakugakubo**
**Kutsukake-cho, Toyoake, Aichi(JP)**
Inventor: **Nagatsu, Toshiharu, c/o Inst.for Comprehensive Med Science,**
**Fujita Health Univ.,**
**1-98 Dengakugakubo**
**Kutsukake-cho, Toyoake, Aichi(JP)**

(74) Representative: **DIEHL GLAESER HILTL & PARTNER**
**Flüggenstrasse 13**
**D-80639 München (DE)**

(54) **Oncopterin diagnostic agent for cancerous diseases, method for the isolation thereof, and substance and method for determining the content of diagnostic agent in liquids.**

(57) Described is 2-(3-aminopropyl)amino-4-hydroxy-6-[(1'R,2'S)-1',2'-dihydroxypropyl] pteridine which can be used as a cancer specific diagnostic agent and therefore is called "oncopterin". Besides, it is referred to a substance for quantitatively determining the diagnostic agent as well as detection and purification methods for the diagnostic agent. The present diagnostic agent is more specific than known cancer indicators and makes possible the routine detection on a micro scale on the basis of any body fluid, particularly urine. Oncopterin can be separated from other pteridines, like biopterin, by means of high pressure liquid chromatography (HPLC).

EP 0 608 570 A1

The invention relates to oncopterin (certain pteridine), i.e., 2-(3-aminopropyl)amino-4-hydroxy-6-[(1'R,2')-dihydroxypropyl]pteridine, a diagnostic agent for cancerogenic diseases, a method for the isolation thereof, and a substance and a method for determining the content of the diagnostic agent in liquids.

Cancer is a disease which often proves fatal and claims an ever larger toll among humans. For this reason, numerous attempts have been made for a long time to develop appropriate diagnostic methods by which cancer diseases can be recognized as early as possible, as in this case the chances of a cure are best.

Dykstra, W. G. and Herbst, E. J. (1965), Spermidine in regenerating liver: relation to rapid synthesis of ribonucleic acid, Science 149, 428 - 429, and Russel, D. and Snyder, S. H. (1968), Amine synthesis in rapidly growing tissues: ornithine decarboxylase activity in regenerating rat liver, chick embryo, and various tumors, Proc. Nat. Acad. Sci. USA 60, 1420 - 1427, already established many years ago that biogenous polyamines, such as putrescine (1,4-diaminobutane), spermidine (N-(3-aminopropyl)-1,4-diaminobutane) and spermine (N,N'-bis-(3-aminopropyl)-1,4-diaminobutane), are synthesized to a larger extent in fast growing tissue, such as cancer tissue, as compared to tissue that grows more slowly.

After Russel, D H. (1971), Increased Polyamine concentrations in the urine of human cancer patients, Nature (New Biol.) 233, 144 - 145, found out for the first time that increased concentrations of biogenous polyamines can be detected in the urine of cancer patients, it has been assumed that the polyamine concentration in patients' body fluids can be used as biochemical markers for the diagnosis of various cancer diseases, e.g., tissue or blood cancer.

Relevant methods were described by Russel, D. H. and Russel, S. D. (1975), Relative usefulness of measuring polylamines in serum, plasma, and urine as biochemical markers of cancer, Clin. Chem. 21, 860 - 863, Bachrach, U., (1976), Polyamines as chemical markers of malignancy, Ital. J. Biochem. 25, 77 - 93, Fujita, K., Nagatsu, T., Maruta, K., Ito, M., Senba, H., and Miki, K. (1976), Urinary putrescine, spermidine, and spermine in human blood and solid cancers and in an experimental gastric tumor of rats, Cancer Res. 36, 1320 - 1324, and Maruta, K., Teradaira, R., Watanabe, N., Nagatsu, T., Asano, M., Yamamoto, K., Matsumoto, T., Shinoya, Y., and Fujita, K. (1989), Simple, sensitive assay of polyamines by high-performance liquid chromatography with electrochemical detection after post-column reaction with immobilized polyamine oxidase, Clin. Chem. 35, 1694 - 1696.

Besides, Nixon, J. C. (1985), "Naturally occurring pterins", in "Folates and Pterins" (Blakley, R. L. and Benkovic. S. J., eds.), Vol. 2, pp. 1 - 42, John Wiley & Sons, New York, and Hausen, A., Fuchs, D., Reibnegger, G., Werner, E. R., and Wachter, H. (1989), Neopterin in clinical use, Pteridines 1, 3 - 10, found that the neopterin level in urine and blood serum of cancer patients had increased. Neopterin has the following chemical structural formula:

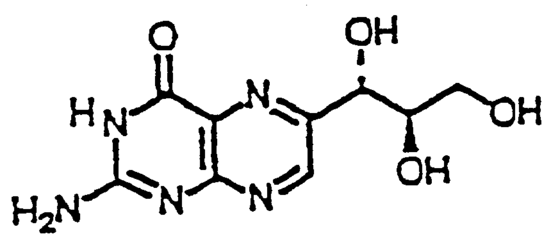

Finally, Rokos, H., Rokos, K., Frisius, H. and Kirstaedter, H. -J. (1980), Altered urinary excretion of pteridines in neoplastic disease, determination of biopterin, neopterin, xanthopterin, and pterin, Clin. Chim. Acta 105, 275 - 286, Stea, B., Halpern, R. M., Halpern, B. C. and Smith, R. A. (1981), Urinary excretion levels of unconjugated pterins in cancer patients and normal individuals, Clin. Chim. Acta 113, 231 - 242, as well as Nagatsu, T., Yamaguchi, T., Sawada, M., Fujita, K., Shinpo, K., Ito, M., Hirano, M., Sugimoto, T., Matsuura, S., and Akino, M. (1984), Elevated levels of polyamines and radioimmunoassayable 6-hydrooxymethylpterin-like compound in urine from cancer patients, Biogenic Amines 1, 51 - 62, report that biopterin and some other pteridines are in some cases present to a higher extent in the body fluids of cancer patients than in healthy individuals. The formula of biopterin is as follows:

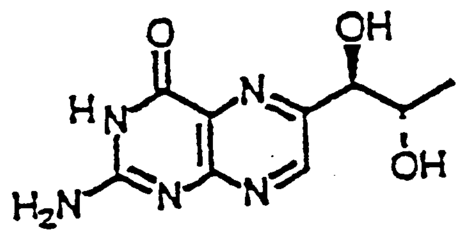

All the afore-mentioned markers and methods have the disadvantage, however, that the isolated substances do not necessarily indicate a cancer disease, as increased amounts of said substances may also be caused by the proliferation of healthy fast-growing tissues.

It is therefore the object of the present invention to state an endogenous marker or indicator for the reliable and specific diagnosis of cancer. Besides, there shall be provided a method for isolation and a substance for quantitative determination of the diagnostic agent. This object is solved by the compounds given in independent claims 1 and 19, the diagnostic agent according to independent claim 2, the substance according to independent claim 7, the methods according to independent claims 8, 14 and 21 and the use according to independent claim 27. Further advantageous features and embodiments, aspects and details of the invention are evident from the dependent claims, the description, the examples and the drawings. The claims are to be understood as a first non-limiting approach to define the invention in general terms.

The present invention provides an endogenous marker or indicator for the reliable and specific diagnosis of cancer.

Besides, the present invention provides a method for isolation and a substance for quantitative determination of the diagnostic agent.

Furthermore, the present invention provides a novel compound which is at least useful for solving the preceding object.

The above object is solved according to a first aspect of the invention by means of a specific compound, i.e., 2-(3-aminopropyl)amino-4-hydroxy-6-[(1'R,2'S)-1',2'-dihydroxypropyl]pteridine, hereinafter referred to as "oncopterin".

According to a second aspect of the invention, there is provided a diagnostic agent for cancerogenic diseases, which is or comprises said oncopterin. Oncopeterin is obtainable by either isolation from the body fluid or by synthesis.

According to a third aspect of the invention, there is provided a specific substance, comprising said oncopterin, for the quantitative determination of oncopterin in a body fluid, preferably urine.

According to fourth and fifth aspects of the invention, there are provided methods for detecting the oncopterin and quantitative determination thereof, respectively.

In the fourth aspect, a sample containing the compound is split up via a reverse phase or cation exchange HPLC column by means of an acidic eluting buffer solution, and the intensity of fluorescence of the eluate is determined, at certain wavelength, preferably of 450 nm.

In the fifth aspect, a concentration of oncopterin is determined in comparison with standard concentration thereof.

According to a sixth aspect of the invention, there is provided a method for isolating the oncopterin from a body fluid, preferably urine, comprising the following steps:

(i) applying a body fluid, preferably a supernatant of centrifuged body fluid, to an anion exchange column, washing the column and eluting the column with an electrolytic solution,

(ii) applying the eluate of step (i) to a cation exchange column, washing the column and eluting the column with an acidic solution, and

(iii) applying the eluate of step (ii) to a reverse phase column and evaporating the eluate of the reverse phase column to dryness.

Oncopterin has the following structural formula:

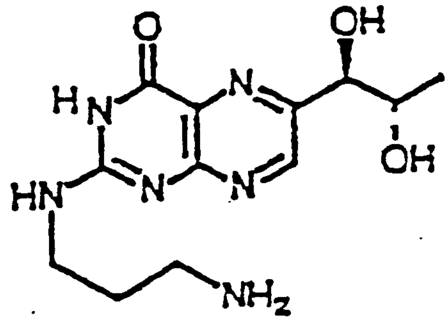

Oncopterin is a biogenous amine whose presence is described for the first time in the following. The compound has a unique structure. It contains both a polyamine residue and the pterin framework structure. Both components have already been used in an isolated manner with moderate success as indicators of cancer diseases of humans. Oncopterin is, for the first time, a compound which combines both indicators. Oncopterin accumulates in cancer patients in all body fluids as compared to healthy individuals; with regard to routine determination of the concentration, it has turned out to be most favorable to determine oncopterin concentration from patients' urine, however.

The chemical relationship with the naturally occurring afore-mentioned substances, neopterin and biopterin, is conspicuous. Nevertheless the present inventors succeeded for the first time to detect oncopterin as naturally occurring substance, particularly in man, to isolate it and to clear up the chemical structure.

Fig. 1 shows various HPLC chromatograms (A to D) of a urine sample of a patient suffering from cancer of the liver,

Fig. 2 shows the UV spectrum of oncopterin acetate in water,

Fig. 3 shows the $^{1}H$ NMR spectrum at 270 MH and 50 °C in $D_2O$ of oncopterin-acetate, each signal being assigned to the proton(s) of the respective carbon atom in the structural formula shown, and

Fig. 4 shows the CD spectrum of oncopterin-acetate in water.

Due to its specificity as an indicator for cancer diseases, oncopterin is an excellent diagnostic agent for cancerous diseases. A particularly high content of said compound is found in the body fluids of patients suffering from a tissue cancer, such as lung cancer, cancer of the liver, bladder cancer, cancer of the prostate and/or ovarian cancer. In the case of blood cancer, such as various kinds of leukemia, the level of oncopterin in the patients' body fluids is higher than that of healthy control persons, too, although not to such an extent as in tissue cancer patients.

The diagnostic agent can be obtained from any body fluid, such as blood, lymph or urine. The easiest way to determine the oncopterin content, without any operative procedure to be performed on the patient, is the determination on the basis of urine; it is therefore particularly preferred that oncopterin be obtained from urine.

In the natural environment and also in isolated form, the diagnostic agent is often present as acid amid. It has therefore proved favorable to use the diagnostic agent in this form.

In order to determine an unknown oncopterin concentration in the body fluid of a human being, a calibrating plot can be used which has been established on the basis of known quantities of the compound. Said known quantities can consist of oncopterin that has been obtained naturally and/or produced synthetically. When establishing the calibrating plot, the values for increasing dilutions of a substance containing oncopterin are determined by way of optical methods. The unknown concentration of an oncopterin-containing liquid can then be easily determined by comparison with the standard concentrations of the calibrating plot.

It was not easy to isolate pure oncopterin from human urine, as, due to the great structural similarities with neopterin and biopterin, the separation from these substances caused problems. However, a method was established by which separation of oncopterin from other pteridines was made possible. Said method was established according to the following examples.

Example 1

80 l urine of patients suffering from a malignant lymphoma was evaporated to a volume of 2 l on a rotary evaporator.

The solution evaporated as mentioned above was then centrifuged to remove a precipitate. 50 ml of the supernatant was applied to a column filled with DEAE (diethylaminoethyl)-Cellulofine having an internal diameter of 10 cm and a length of 50 cm, which had been obtained from Seikagaku Corp. The column was then washed with water until the eluate became neutral. Thereafter, the column was eluted with 2.8 % ammonia (3.5 l), and the eluate was evaporated to a volume of about 100 ml on a rotary evaporator. The solution evaporated as mentioned above was then applied to a column filled with CM (carboxymethyl)-Cellulofine having an internal diameter of 10 cm and a length of 50 cm, which had been obtained from Seikagaku Corp. The column was then washed with 3 l water and subsequently eluted with 3 l of 3 % formic acid. The rest of the aforementioned supernatant was fractionated accordingly.

The solutions eluted by formic acid from the latter column were combined and evaporated to dryness on a rotary evaporator. The residue was dissolved in 50 ml water and applied to a Florisil (magnesium silicate) column (internal diameter 2 cm, length 40 cm, obtained from Wako Pure Chemical Industries) in two 25 ml portions. The column, after being washed with 1.5 l water, was eluted by 600 ml of 2.8 % ammonia. The solutions eluted by ammonia were combined. The two combined eluates of the two portions were evaporated to dryness. The residue was absorbed in 24 ml water, and 8 ml portions thereof were subjected to chromatography at medium pressure on an ODS CQ-3 column (internal diameter 5 cm, length 45 cm, obtained from Fuji Gel Corp.), 2 % acetic acid being used as eluting agent. This procedure was repeated 3 times more to fractionate the remaining part of the solution. From the aforementioned eluate, 4.9 mg oncopterin was recovered in the form of acetate by means of subsequent preparative HPLC (high performance liquid chromatography) via an Inertsil PREP-ODS column (internal diameter 20 mm, length 250 mm; GL Sciences, Inc.) eluted by water and subsequent evaporation of the eluate.

Further, an analytic HPLC was carried out by means of the JASCO 880-PU device equipped with a JASCO FP-210 fluorescence detector (excitation at 355 nm, emission at 450 nm) and an SIC chromatocorder 12. Separation was effected via a Develosil ODS-K-5 reverse phase column (internal diameter 4.6 mm, length 250 mm; Nomura Chemicals) eluted with a solution of 30 mM ammonium phosphate, pH-value 3.5, containing 2 % methanol, or on a Nucleosil 10 SA strong cation exchange column (internal diameter 4.6 mm, length 250 mm; Chemco Scientific Co., Ltd.) eluted with a solution of 30 mM ammonium phosphate, pH-value 3.5, containing 10 % methanol.

The flow velocity was 1 ml/min for both columns, respectively.

Example 2

We have found that oncopterin concentrations in urine increase after acid hydrolysis, like those of polyamines. Therefore, the total oncopterin concentrations in urine were measured after acid hydrolysis. The concentrations of biopterin were also measured at the same time, as described below.

HPLC analysis of oncopterin and biopterin in urine was carried out with a JASCO 880-PU chromatograph, equipped with a JASCO FP-210 fluorescence detector (excitation at 355 nm, emission at 450 nm) and a SIC Chromatocorder 12, on a Develosil K-5 reverse-phase column (internal diameter 4.6, length 250 mm; Nomura Chemicals Co., Ltd.), eluted with a solution of 30 mM ammonium phosphate, pH value 3.5 (1.0 ml/min), or on a Nucleosil 100-5SA strong cation exchange column (internal diameter 4.6, length 150 mm; Nagel), eluted with a solution of 100 mM ammonium phosphate , pH value 3.5, containing 10 % methanol (1.0 ml/min).

The retention volumes of oncopterin and biopterin from urine samples were 30.79 ml and 29.96 ml for the Develosil K-5 column, and 10.61 ml and 3.45 ml for the Nucleosil 100-5SA column.

Oncopterin in the present urine samples was confirmed by direct comparison with authentic oncopterin isolated from urine as shown in Fig. 1. The concentration of creatinine in urine was determined according to the reported method (by Boutwell, J. H. (1961) Clinical Chemistry, Laboratory Manual and Methods, pp. 184-188, Lea & Febiger, Philadelphia).

See Fig. 1; samples A and B were obtained on a Develosil K-5 column, samples C and D were obtained on a Nucleosil 100-5SA column. In the samples B and D, 10 pmol standard oncopterin was added to the 5 µl urine sample. The oncopterin and biopterin concentrations in these samples were 254 µmol/mol creatinine and 50 µmol/mol creatinine, respectively.

Example 3

As a matter of routine, the oncopterin concentrations were determined as follows. The first morning urine from patients suffering from various kinds of cancer and from healthy control persons were deep-frozen immediately after collection and stored at -80 °C in the dark until it was measured. As it had been

found that the oncopterin concentration in urine increases after acid hydrolysis, the individual samples were subjected to acid treatment prior to determination. For this purpose, a mixture of urine (120 μl) and 6 M hydrochloric acid (60 μl) was heated for two hours at 100 °C in a glass tube sealed by a rubber stopper. The solution was then lyophilized. The residue was mixed well and absorbed in 120 μl of a 30 mM ammonium phosphate buffer solution, pH value 3.5, and separated by centrifugation for 10 minutes at 3000 revolutions per minute. A 5 μl aliquot of the supernatant, which corresponded to 5 μl urine, was used in the afore-described HPLC analysis.

Without the hydrolysis step, oncopterin could be hardly detected in the urine of healthy persons (controls). The concentrations of this compound in the nonhydrolyzed urine of cancer patients were lower than those in hydrolyzed urine. The detection limit for oncopterin lies at 0.2 pmol/5 μl urine in the above-described method. Hence, there has been established a highly sensitive microprocess for the routine determination of oncopterin in body fluid.

The oncopterin concentrations detected in the urine of a control group of healthy persons and patients with various cancer diseases are indicated in Table 1. It is conspicuous that patients suffering from solid cancers (cancers of relatively firm tissues), e.g., hepatomas, prostatic cancer and bladder cancer, have statistically significant increases in oncopterin concentrations in urine. Patients suffering from blood cancers, e.g., myelomas, lymphomas and acute myelocytic leukemia, had lower oncopterin concentrations in their urine; however, the concentrations were still considerably higher than those of the control group. A reason for the relatively large scattering of values within a group might be due to the different clinical conditions of the cancer patients.

Although the natural way (biogenic pathway) of synthesis of oncopterin is not known, it was assumed that biopterin might be a possible precursor of oncopterin in metabolism. For this reason, the ratio of concentrations of the two substances was investigated, as indicated in Table 1, too. It was found, however, that the concentrations of biopterin in urine were only higher than those of a healthy control group in the case of lymphomas and acute myelocytic leukemias. Otherwise, no significant relationship between the two compounds in the urine of cancer patients could be found.

Besides, it was reported that neopterin played a role in the cellular immune response and could therefore serve as an indicator of the state of activation of the immune system, cf. the article by Hausen et al mentioned in the beginning, as well as Huber, C., Batchelor, J. R., Fuchs, D., Hausen, A., Lang, A., Niederwieser, D., Reibnegger, G., Swetly, P., Troppmair, J., Wachter, H. (1984) in J. Exp. Med. 160, 310 - 316.

It was also reported that the cancer patients exhibited an increased neopterin concentration; cf. the articles by Nixan and Hausen et al mentioned in the beginning, as well as Ogiwara, S., Kiuchi, K., Nagatsu, T., Teradaira, R., Nagatsu, L., Fujita, K., & Sugimoto, T. (1992) in Clin. Chem., presently being in the press.

However, no significant relationship between the oncopterin and neopterin concentrations in urine was found.

As mentioned in the afore-said literatures, the concentrations of polyamines (spermidine, spermine) and neopterin in the urine of various cancer patients are 2 to 10 times and 2 to 8 times higher, respectively. With the newly found compound, oncopterin, patients suffering from a cancer of a "solid" tissue have an oncopterin concentration in their urine that is about 70 to 100 times higher than that of healthy persons. Hence, oncopterin represents a more reliable indicator of cancer diseases than the markers previously used.

Measurements

In order to clear up the structure of the substance obtained (oncopterin), NMR (nuclear magnetic resonance) spectroscopy was employed. The $^{1}H$ NMR spectrum was determined in $D_2O$ at 50 °C on a JEOL JNM-EX 270 spectrometer. The chemical shifts correspond to the proton at 4.50 ppm in the solvent. The UV spectrum of the substance was determined in water by a Hitachi 220 A spectrophotometer. The CD (circular dichroism) spectrum was recorded by a JASCO J-500 spectropolarimeter in water.

As already indicated. in the beginning, the separation of oncopterin from pteridines which also occur naturally for example, such as neopterin and biopterin, is very difficult on account of the chemical similarity of the substances. Hence, the purification step via the DEAE column does not yet lead to separation, either. In this manner, only other substances present in the starting fluid can be separated from the pteridines. Separation of the pteridines was only effected via the CM column, as neopterin and biopterin, which are not so basic, could be eluted by water, whereas the stronger base oncopterin could not be separated from the column by water. This way only effected with the aid of 3 % formic acid.

The UV spectrum of the substance obtained as shown in Fig. 2 showed maximum values at 218, 243, 279 and 349 nm (log $\epsilon$ 4.25, 4.18, 4.35 and 3.91). This spectrum shows a large conformity with the spectra of neopterin and biopterin, which shows that all three compounds have the same chromophorous group, i.e., 2-amino-4-hydroxypteridine. As it specifically occurs in cancer patients, the substance, whose structure had first not been completely clarified, was called oncopterin.

In the $^1H$ NMR spectrum of oncopterin as shown in Fig. 3, the singlet at 8.71 ppm (signal a; 1 H) was assigned to the proton located at the pteridine ring, and the other singlet at 1.97 ppm (signal h; 3 H) was assigned to the acetyl group of the acetate anion. The three signals at 4.77 ppm (signal b; 1 H, d, J = 4.6 Hz), 4.13 - 4.22 ppm (signal c; 1 H, m) and 1.19 ppm (signal d; 3 H, d, J = 6.3 Hz) were assigned to the 1,2-dihydroxypropyl group. The other three signals at 3.10 ppm (signals; 2 H, t, J = 6.9 Hz), 1.95 - 2.05 ppm (signal f; 2 H, m) and 3.56 ppm (signal g; 2 H, t, J = 6.6 Hz) indicated the presence of a trimethylene group.

From these spectra and data as well as the strongly basic properties of the substance, it has been concluded that oncopterin is a stereoisomer of the $N^2$-(3-aminopropyl)biopterin, i.e., 2-(3-aminopropyl)-amino-4-hydroxy-6-[(1'R,2'S)-1',2'-dihydroxypropyl]pteridine. This assumption was confirmed by the direct comparison of the synthetic compound (see Sawada, M., Yamaguchi, T., Sugimoto, T. Matsuura, S., and Nagatsu, T. (1984) Polarization fluoroimmunoassay of biopterin and neopterin in human urine, Clin. Chem. Acta 138, 275 - 282) with the isolated compound. In an HPLC run in Example 1, the retention volume for both compounds was 18.42 ml on the Develosil ODS-K-5 column and 63.93 ml on the Nucleosil 10 SA column. The assumption was also bolstered by a comparison of the $^1H$ NMR and UV spectra. Finally, it was also found that the 1'2'-dihydroxypropyl side chain of the oncopterin had the (1'R,2'S) configuration, i.e., the L-erythro configuration, as both the synthetic and the isolated compounds had the same CD spectra (see Fig. 4; $\lambda_{max}$ (nm) ($\Delta\epsilon$): 380(-0.04), 333(-0.36), 289(-0.20) 269(-0.81) 259(-0.47), 242(-1.50) and 221(+3.00); concentration: $8.4 \times 10^{-5}$ mol/l in water.

To sum up, it can be said that oncopterin is a highly specific diagnostic agent for cancer diseases, which can be detected quickly and sensitively by the afore-described method. Due to the fact that detection is possible on the micro scale and no expensive reagents and complicated apparatus are necessary, the present determination method is excellently suited for routine application in cancer diagnostics.

**Table 1**

Oncopterin and biopterin concentrations in the urine of a healthy control group and of persons suffering from various types of cancer

| | Number of samples | average age | oncopterin (µmol/mol creatinin) mean value | ± standard deviation | biopterin (µmol/mol creatinin) mean value | ± standard deviation |
|---|---|---|---|---|---|---|
| control group | (10) | 27,2 | 1,7 | ± 1,7 | 256,6 | ± 48,5 |
| lung cancer | (11) | 67,9 | 51,6 | ± 27,6 | 139,4 | ± 37,5 |
| cancer of the liver | (10) | 63,1 | 118,7 | ± 35,8* | 182,2 | ± 51,3 |
| cancer of the prostate | (5) | 73,4 | 197,6 | ± 91,5* | 159,4 | ± 55,1 |
| bladder cancer | (7) | 61,3 | 132,6 | ± 72,0* | 372,7 | ± 129,6 |
| ovarian cancer | (6) | 50,8 | 46,6 | ± 46,7 | 197,0 | ± 71,5 |
| myeloma | (10) | 57,4 | 9,4 | ± 14,5 | 260,7 | ± 69,9 |
| acute myelocytic leukemia | (11) | 40,5 | 4,2 | ± 4,2 | 513,3 | ± 89,9* |
| lymphoma | (15) | 50,1 | 17,2 | ± 10,6 | 519,1 | ± 83,1* |

* significantly increased as compared to normal control persons ($P < 0,05$)

## Claims

1. 2-(3-aminopropyl)amino-4-hydroxy-6-[(1'R,2'S)-1',2'-dihydroxypropyl] pteridine.

2. Diagnostic agent for cancerous diseases, characterized in that it is 2-(3-aminopropyl) amino-4-hydroxy-6-[(1'R,2'S)-1',2'-dihydroxypropyl] pteridine.

3. Diagnostic agent according to claim 2, characterized in that it is present as acid amide.

4. Diagnostic agent according to claim 2 or 3, characterized in that it is obtained from body fluid, preferably urine.

5. Diagnostic agent according to claim 2 or 3, characterized in that it is obtained synthetically.

6. Diagnostic agent according to any one of claims 2 to 5, characterized in that the cancerous disease is a tissue cancer, especially lung cancer, cancer of the liver, cancer of the prostate, bladder cancer or ovarian cancer.

7. Substance, comprising 2-(3-aminopropyl)amino-4-hydroxy-6-[(1'R,2'S)-1',2'-dihydroxypropyl] pteridine for the quantitative determination of said pteridine in a body fluid, preferably urine.

8. Method for detecting the compound 2-(3-aminopropyl)amino-4-hydroxy-6-[(1'R,2'S)-1',2'-dihydroxypropyl] pteridine in a liquid, characterized in that a sample containing the compound is split up via a reverse phase or cation exchange HPLC column by means of an acidic eluting buffer solution, and the intensity of fluorescence of the eluate is determined, preferably at a wavelength of 450 nm.

9. Method according to claim 8, characterized in that the sample was hydrolized with acid prior to measurement.

10. Method according to claim 9, characterized in that hydrolysis is effected with 6 M hydrochloric acid for 2 hours at 100 °C.

11. Method according to any one of claims 8 to 10, characterized in that the fluid is urine.

12. Method according to claim 11, characterized in that the urine is from a patient suffering from cancer.

13. Method according to claim 12, characterized in that the cancer is a tissue cancer, especially lung cancer, cancer of the liver, cancer of the prostate, bladder cancer or ovarian cancer.

14. Method for the quantitative determination of the compound 2-(3-aminopropyl)amino-4-hydroxy-6-[-(1'R,2'S)-1',2'-dihydroxypropyl] pteridine, characterized in that a concentration is determined in comparison with one standard concentrations.

15. Method according to claim 14, characterized in that the compound has been obtained according to one of the methods described in claims 8 to 13.

16. Method for isolating 2-(3-aminopropyl)amino-4-hydroxy-6-[(1'R,2'S)-1',2'-dihydroxypropyl] pterdine from a body fluid, comprising the following steps:
(i) applying a body fluid, preferably a supernatant of centrifuged body fluid, to an anion exchange column, washing the column and eluting the column with an electrolytic solution,
(ii) applying the eluate of step (i) to a cation exchange column, washing the column and eluting the column with an acidic solution, and
(iii) applying the eluate of step (ii) to a reverse phase column and evaporating the eluate of the reverse phase column to dryness.

17. Method according to claim 16, wherein the body fluid, which is preferably urine, is from a cancer patient.

18. Method according to claim 17, characterized in that the cancer is a tissue cancer, especially cancer of the liver, lung cancer, cancer of the prostate, bladder cancer or ovarian cancer.

**19.** 2-(3-aminopropyl)amino-4-hydroxy-6-[(1'R,2'S)-1',2'-dihydroxypropyl] pteridine, obtainable by the steps (i) to (iii) of claim 16.

**20.** Compound according to claim 19, characterized in that the body fluid is urine, which especially comes from a cancer patient.

**21.** Method for detecting cancer cells, characterized in that the concentration of the compound 2-(3-aminopropyl)amino-4-hydroxy-6-[(1'R,2'S)-1',2'-dihydroxypropyl] pteridine in a body fluid is determined.

**22.** Method according to claim 21, characterized in that the compound was obtained according to one of the methods described in claims 16 to 18.

**23.** Method according to claim 21 or 22, characterized in that the sample is hydrolized with acid prior to the determination of concentration.

**24.** Method according to claim 23, characterized in that hydrolysis is carried out with 6 M HCl for 2 hours at 100 °C.

**25.** Method according to any one of claims 21 to 24, characterized in that the method is carried out on a micro scale.

**26.** Method according to any one of claims 21 to 25, characterized in that the cancer cells are from a tissue cancer, especially cancer of the liver, lung cancer, cancer of the prostate, bladder cancer or ovarian cancer.

**27.** Use of the compound 2-(3-aminopropyl)amino-4-hydroxy-6-[(1'R,2'S)-1',2'-dihydroxypropyl] pteridine as an indicator for a cancer disease.

FIG.1 (A)

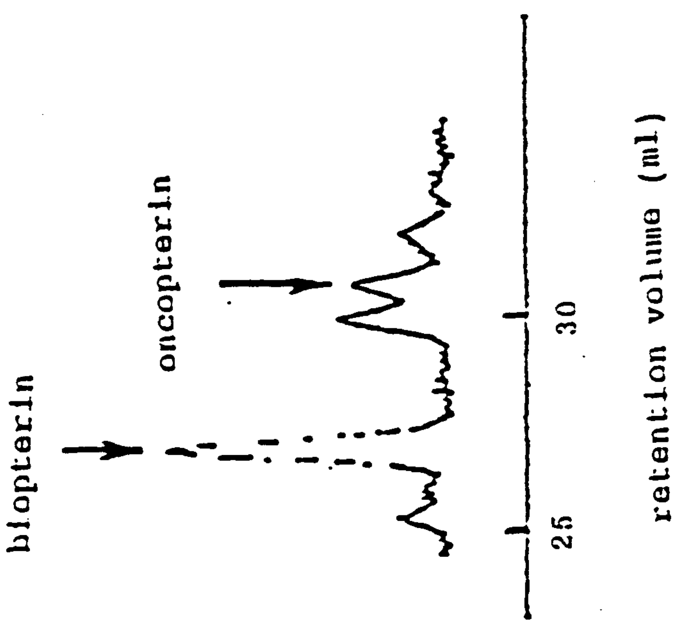
biopterin
oncopterin
25
30
retention volume (ml)

FIG.1(C)

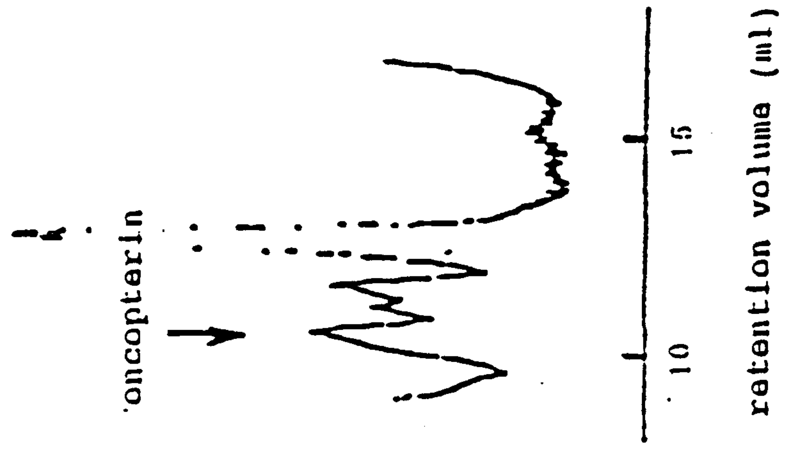
oncopterin
10
15
retention volume (ml)

FIG.1(B)

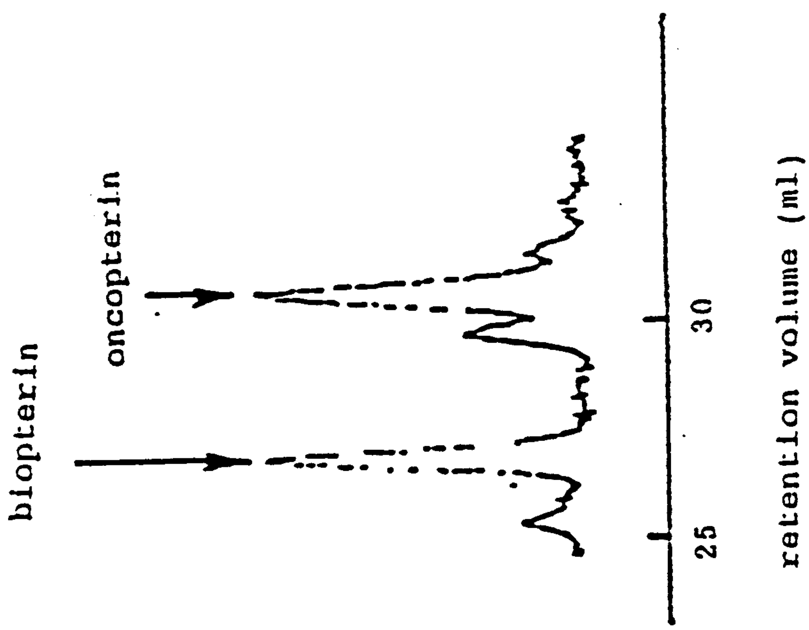
biopterin
oncopterin
25
30
retention volume (ml)

FIG.1(D)

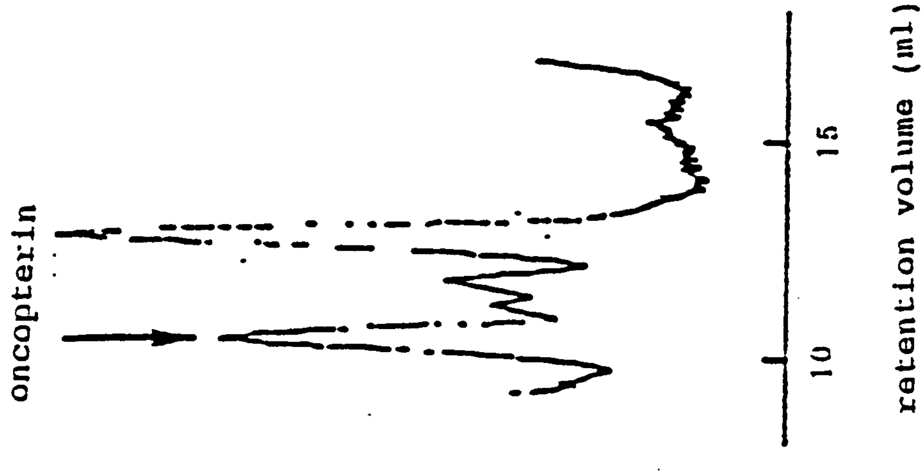
oncopterin
10
15
retention volume (ml)

FIG.2

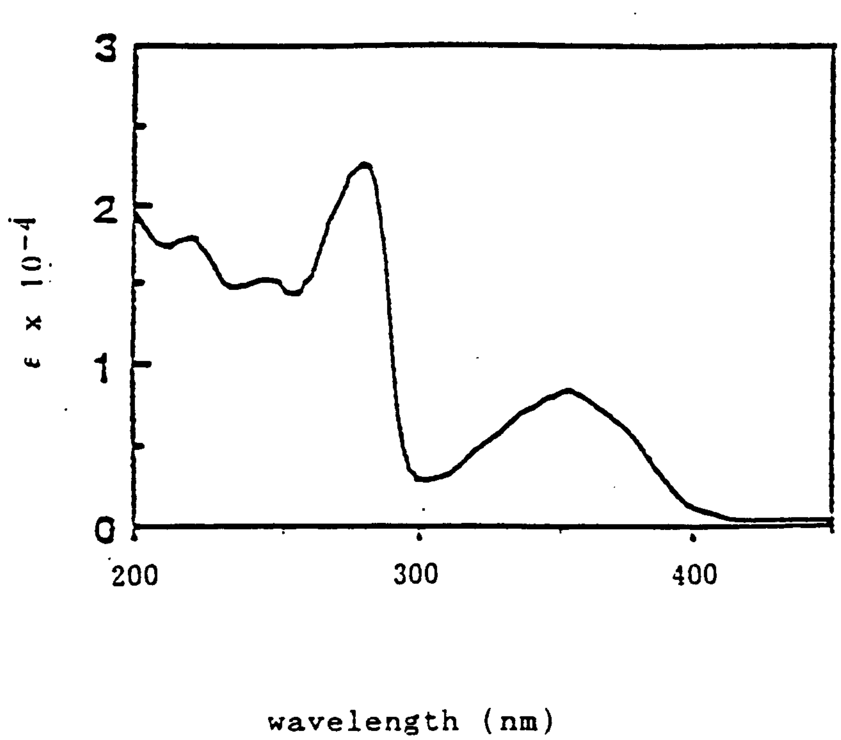

FIG. 3

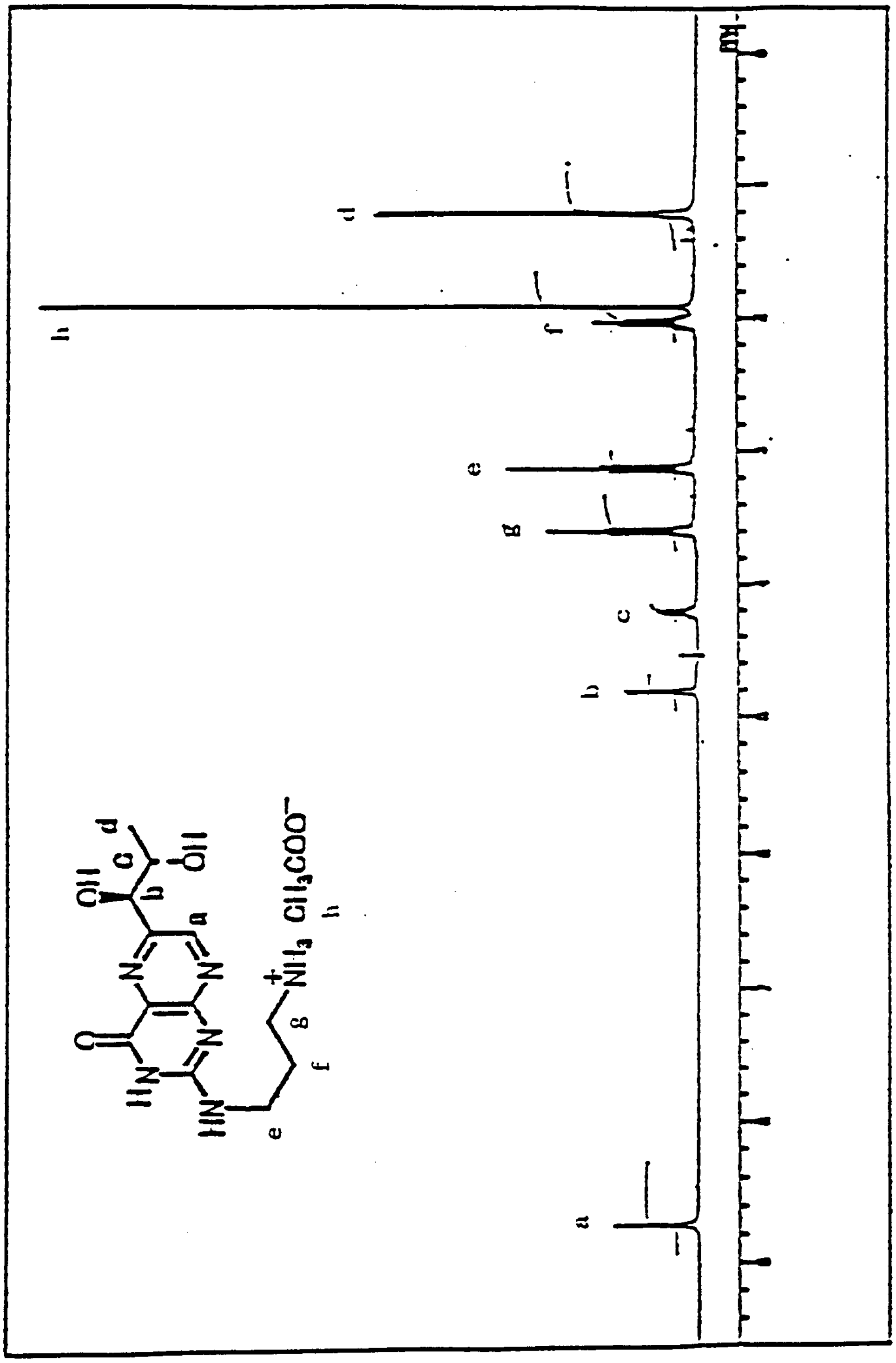

FIG.4

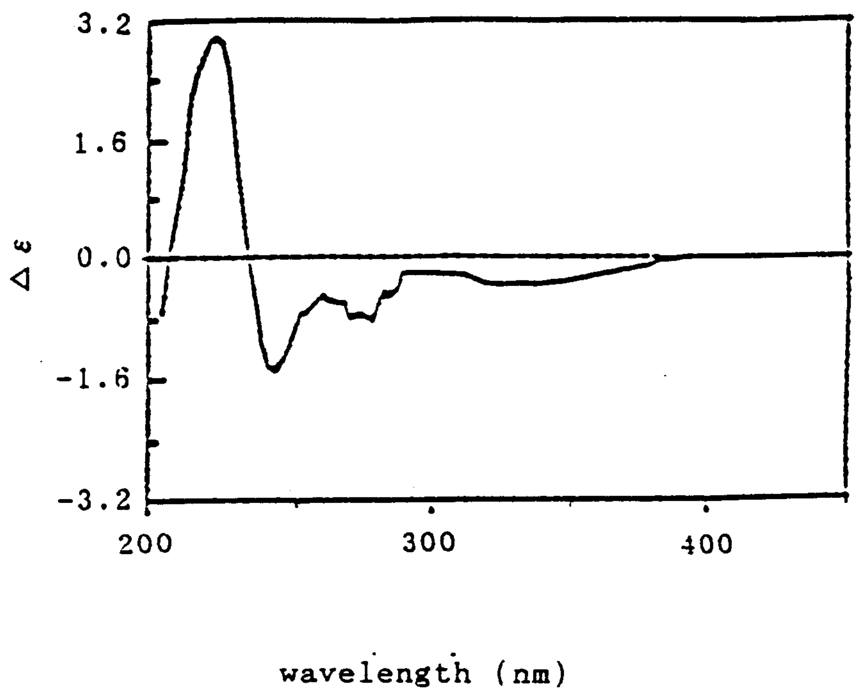

European Patent Office

# EUROPEAN SEARCH REPORT

Application Number
EP 93 12 1138

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.5) |
| A | EP-A-0 012 444 (HENNING BERLIN GMBH)<br>* the whole document *<br>--- | 1,2,7,8,21 | C07D475/04<br>G01N33/50 |
| A | TETRAHEDRON LETTERS,<br>vol.33, no.10, 1992, GREAT BRITAIN<br>pages 1341 - 1342<br>SHOJI OGIWARA ET AL. 'Occurence of Umanopterin, a new Diastereomer of Neopterin, in urine from cancer patients'<br>* the whole document *<br>--- | 1,8,21 | |
| A,D | CLINICA CHIMICA ACTA,<br>vol.105, no.2, 1980, AMSTERDAM<br>pages 275 - 286<br>H. ROKOS ET AL. 'Altered Urinary excretion of pteridines in neoplastic Disease.........'<br>* the whole document *<br>--- | 1,8,14,21 | |
| A,D | CLINICA CHIMICA ACTA,<br>vol.113, no.1, 1981, AMSTERDAM<br>pages 231 - 242<br>BALDASARRE STEA ET AL. 'Urinary excretion levels of unconjugated pterins in cancer patients.....'<br>* the whole document *<br>----- | 1,7,21 | TECHNICAL FIELDS SEARCHED (Int.Cl.5)<br>C07D<br>G01N |

The present search report has been drawn up for all claims

| Place of search | Date of completion of the search | Examiner |
|---|---|---|
| BERLIN | 21 April 1994 | Kyriakakou, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document